# EUROPEAN PATENT APPLICATION

(11) **EP 4 266 854 A1**
(43) Date of publication of application: **25.10.2023**
(21) Application number: 22908824.0
(22) Date of filing: 03.03.2022
(51) Int. Cl.: H10K 71/00, H10K 85/00, C07C 43/21

(54) **FULLERENE DERIVATIVE AND PEROVSKITE SOLAR CELL**

(71) Applicant: Contemporary Amperex Technology Co., Limited, Ningde City, Fujian 352100 (CN)
(72) Inventor: LIANG, Weifeng, Ningde, Fujian 352100 (CN); HUANG, Zhihan, Ningde, Fujian 352100 (CN); CHEN, Changsong, Ningde, Fujian 352100 (CN); GUO, Yongsheng, Ningde, Fujian 352100 (CN); CHEN, Guodong, Ningde, Fujian 352100 (CN)
(74) Representative: Jacob, Reuben Ellis
(86) International application number: PCT/CN2022/079030
(87) International publication number: WO 2023/164877

(57) **Abstract**

The present application provides a fullerene derivative having a C60 fullerene group and a group of a compound of formula (1), formula (2) and/or formula (3) attached thereto, wherein the structural formulas of the compounds of formula (1), formula (2), and formula (3) are as follows: 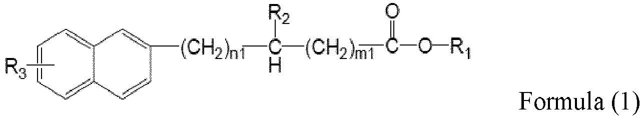 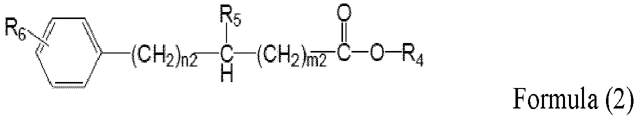 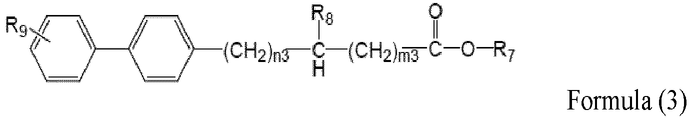 wherein R₁, R₂, R₃, R₄, R₅, R₆, R₇, R₈, R₉, n1, n2, n3, m1, m2, and m3 are as defined in the specification. When using as an electron transport layer in perovskite solar batteries, the fullerene derivatives described in the present application can improve the photon-to-electron conversion efficiency and stability of perovskite solar batteries.

## Description

### TECHNICAL FIELD

The present application relates to a fullerene derivative. Furthermore, the present application also relates to a perovskite solar battery comprising the fullerene derivative.

### BACKGROUND

With the rapid development of the new energy field, perovskite solar batteries are favored due to their advantages such as high photon-to-electron conversion efficiency, simple manufacturing process, low production cost and material cost. Fullerene materials are often used as electron transport layers in perovskite solar batteries due to their properties such as high electron mobility, adjustable energy levels, and low-temperature film formation.

However, there are still many problems in the application of current fullerene materials in perovskite solar batteries, such as poor performance and poor stability of solar batteries prepared therefrom. Therefore, further improvements to solar batteries are still required.

### SUMMARY

The present application is conducted in view of the above subject matter and aims to provide a fullerene derivative useful for both the interface passivation and the electron transport that can improve the photon-to-electron conversion efficiency and stability of a solar battery.

In order to achieve the above purpose, the present application provides a fullerene derivative having a C60 fullerene group and a group of a compound of formula (1), formula (2) and/or formula (3) attached thereto, wherein the structural formulas of the compounds of formula (1), formula (2), and formula (3) are as follows: 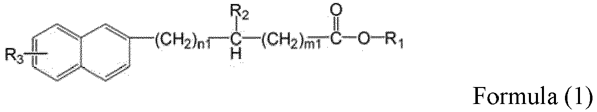 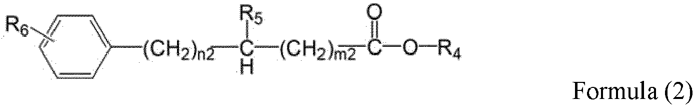 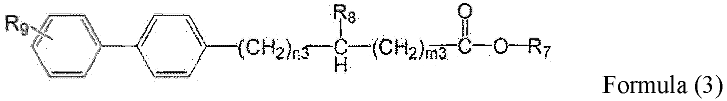 wherein
R₁, R₄, and R₇ are each independently selected from R', a phenyl group, a naphthyl group, a biphenyl group, R' substituted with a halogen, a phenyl group substituted with a halogen, a naphthyl group substituted with a halogen, a biphenyl group substituted with a halogen, a nitrogen-containing group, R' substituted with a nitrogen-containing group, a phenyl group substituted with a nitrogen-containing group, a naphthyl group substituted with a nitrogen-containing group or a biphenyl group substituted with a nitrogen-containing group;
R₂, R₅, and R₈ are each independently selected from hydrogen or R';
R₃, R₆, and R₉ are each independently selected from hydrogen, halogen, -O-R, a nitrogen-containing group, -OH, -OR, -NHCOR, -OCOR, -R, -CH₂COOH, a phenyl group or a naphthyl group;
the nitrogen-containing group is -N(R)₂, -NHR, -NH₂, a trimethylamine group, a triethylamine group or a tripropylamine group;
R' is an alkyl group with 1-5 carbon atoms;
R is an alkyl group with 1-10 carbon atoms;
n1, n2, n3, m1, m2, and m3 are each independently an integer in the range of 0-10, optionally an integer in the range of 0-5, and n1 + m1 ≤ 10, n2 + m2 ≤ 10, and n3 + m3 ≤ 10.

The fullerene derivatives obtained by introducing one or more specific functional groups (e.g., halogens, nitrogen-containing groups, aryl groups, etc.) to PCBM ([6,6]-phenyl-C61-butyric acid methyl ester) can stabilize the phase stability of the perovskite and passivate the interfacial defects of the perovskite, thereby improving the photon-to-electron conversion efficiency and stability of the perovskite solar battery. Without being bound by any theory, it is speculated that the stabilizing effect of perovskite solar batteries and the improvement of the photon-to-electron conversion efficiency of the batteries by these functional groups may be attributed to the fact that these introduced functional groups can increase the binding energy to the proton at position A in perovskite (chemical formula: ABX₃) and can be better anchored with lead, if present, thereby increasing the phase stability of the perovskite. In addition, they can reduce the non-radiative recombination, so that the interfacial defects of the perovskite can be passivated. Different functional groups have different effects when introduced into different positions. For example, the introduction of certain electron-withdrawing functional groups (such as halogens) can passivate interfacial defects on the perovskite surface, and the introduction of certain electron-donating functional groups (such as nitrogen-containing groups and aryl groups (for example phenyl group, naphthyl group)) can adjust the LUMO energy level and HOMO energy level of perovskite.

Thus, the fullerene derivatives described in the present application can improve the photon-to-electron conversion efficiency and stability of perovskite solar batteries.

In any of embodiments, in formula (1) and formula (2),
R₁ is selected from R';
R₂ and R₅ are hydrogen;
R₃ is hydrogen;
R₄ is selected from R', a phenyl group or a trimethylamine group;
R₆ is selected from hydrogen or halogen, optionally hydrogen or fluorine;
n1 + m1 ≤ 5; and
n2 + m2 ≤ 5.

In any of embodiments, in formula (1), formula (2), and formula (3), each C60 fullerene group is attached to 1-3, optionally 2, groups selected from the groups of compounds of formula (1), formula (2) and/or formula (3).

The number of the groups of a compound of formula (1), formula (2) and/or formula (3) attached to the C60 fullerene group may affect the energy level position of the fullerene. As this number increases, the LUMO energy level decreases. However, if this number increases further, the steric hindrance will be increased, which is not conducive to the accumulation of fullerene derivatives, and will reduce the electron transport ability, thereby reducing long-term stability of perovskite solar batteries. Therefore, each C60 fullerene group is optionally attached to 1-3, optionally 2, groups selected from the groups of compounds of formula (1), formula (2) and/or formula (3).

In any of embodiments, the LUMO energy level of the fullerene derivatives ranges from -4.02 to -3.72 eV, optionally from -4.02 to -3.82 eV, and more optionally from -4 to -3.92 eV.

Although the HOMO and LUMO energy levels of the fullerene derivatives are mainly provided by C60, the introduction of the above-mentioned specific functional groups will reduce the LUMO energy levels of the fullerene derivatives described in the present application to better match the energy levels of the conduction band minimum of the perovskite. Thus, the electrons are more easily separated at the interface between the electron transport layer and the perovskite layer made of the fullerene derivatives, reducing the recombination of carriers at the interface, and thus improving the photon-to-electron conversion efficiency of the perovskite solar batteries.

In any of embodiments, the fullerene derivative can have the structure of formula (4), formula (5), formula (6), formula (7), formula (8), formula (9), formula (10), formula (11) or formula (12): 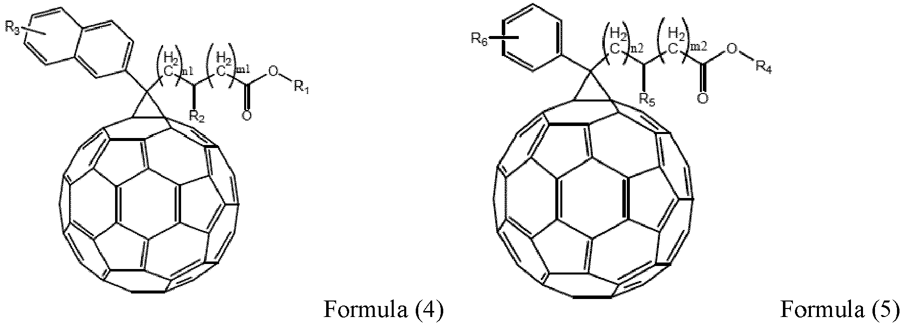 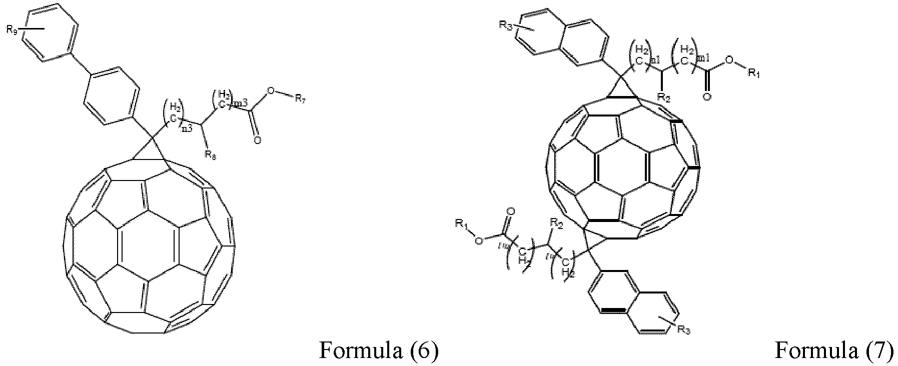 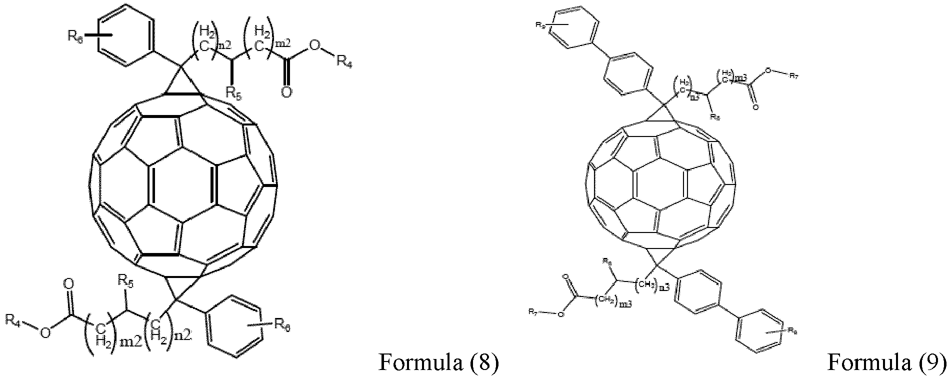 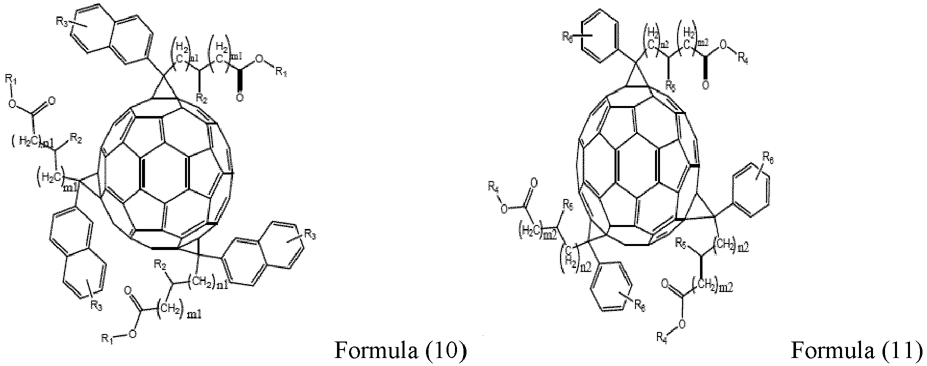 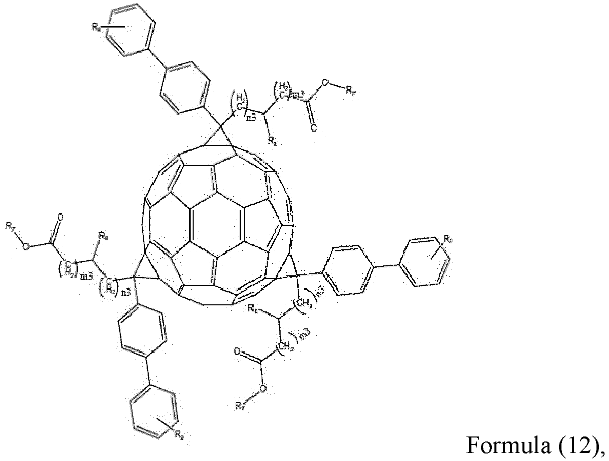 wherein R₁ to R₉, n1 to n3, and m1 to m3 are as defined in formulas (1), (2) and (3).

Among the above structural formulas, formula (4), formula (7), and formula (10) are those in which one, two, and three compounds of formula (1) are respectively attached to C60 fullerene; formula (5), formula (8), and formula (11) are those in which one, two, and three compounds of formula (2) are respectively attached to C60 fullerene; and formula (6), formula (9), and formula (12) are those in which one, two, and three compounds of formula (3) are respectively attached to C60 fullerene.

The fullerene derivatives described in the present application can be prepared by methods similar to those used for the preparation of PCBM, for example, by methods similar to those described in the research paper "Synthesis and purification process of PCBM" documented in the Journal of Chemical Engineering, Vol. 66, No. S2 (Aug. 2015).

Alternatively, the method for preparing the fullerene derivatives described in the present application may comprise the following steps of:
(1) obtaining the precursor 1 of the formula (1), (2) or (3) using a synthetic method known in the art or through commercial purchase, the precursor 1 being similar in structure to formula (1), (2) or (3), differing only in that the aliphatic carbon connected to the benzene or naphthalene ring in the precursor 1 is double-bonded to an oxygen atom to form a carbonyl group;
(2) reacting the precursor 1 with a compound containing sulfonyl hydrazide (optionally p-toluenesulfonyl hydrazide) to synthesize the precursor 2, the precursor 2 being similar in structure to formula (1), (2) or (3), differing only in that the aliphatic carbon connected to the benzene or naphthalene ring in the precursor 2 is double-bonded with a sulfonyl hydrazone (optionally benzenesulfonyl hydrazone); optionally, the reaction comprising the following steps of: dissolving the precursor 1 and the compound containing sulfonyl hydrazide (optionally p-toluenesulfonyl hydrazide) in a solvent, heating to reflux for 2-15 h, then cooling, subsequently storing in the dark for 6-20 h, and then placing at -5°C to -20°C for 12-48 h to obtain the precursor 2; and
(3) reacting the precursor 2 with C60 fullerene to obtain the fullerene derivative described in the present application; optionally, the reaction comprising the following steps of:
   A. mixing the precursor 2 with sodium methoxide and then dissolving in pyridine, withdrawing the air from the reaction system and charging it with an inert gas (such as nitrogen) so as to remove most of the moisture and oxygen in the reactor chamber and the reaction system;
   B. dissolving the C60 fullerene in a solvent (such as o-dichlorobenzene), then adding the obtained C60 fullerene solution dropwise to the above reaction system, and allowing the reaction system to react at 50-90°C for 12-32 h after the dropwise addition; after completing the reaction, removing the solvent, and applying the product as obtained directly to the next step without purification; and
   C. dissolving the product obtained in step B in a solvent (such as o-dichlorobenzene), reacting at 150-210°C for 14-34 h, then removing most of the solvent, and purifying through a silica gel chromatography column using toluene as the mobile phase to obtain the fullerene derivatives described in the present application.

A second aspect of the present application provides a perovskite solar battery, which comprises a conductive glass, a hole transport layer, a perovskite layer, an electron transport layer comprising a fullerene derivative and a back electrode, the fullerene derivative having a C60 fullerene group and a group of a compound of formula (1), formula (2) and/or formula (3) attached thereto, wherein the structural formulas of the compounds of formula (1), formula (2), and formula (3) are as follows: 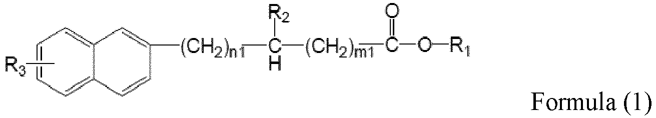 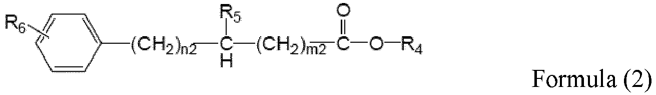 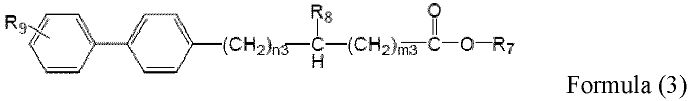 wherein
R₁, R₄, and R₇ are each independently selected from R', a phenyl group, a naphthyl group, a biphenyl group, R' substituted with a halogen, a phenyl group substituted with a halogen, a naphthyl group substituted with a halogen, a biphenyl group substituted with a halogen, a nitrogen-containing group, R' substituted with a nitrogen-containing group, a phenyl group substituted with a nitrogen-containing group, a naphthyl group substituted with a nitrogen-containing group or a biphenyl group substituted with a nitrogen-containing group;
R₂, R₅, and R₈ are each independently selected from hydrogen or R';
R₃, R₆, and R₉ are each independently selected from hydrogen, halogen, -O-R, a nitrogen-containing group, -OH, -OR, -NHCOR, -OCOR, -R, -CH₂COOH, a phenyl group or a naphthyl group;
the nitrogen-containing group is -N(R)₂, -NHR, -NH₂, a trimethylamine group, a triethylamine group or a tripropylamine group;
R' is an alkyl group with 1-5 carbon atoms;
R is an alkyl group with 1-10 carbon atoms;
n1, n2, n3, m1, m2, and m3 are each independently an integer in the range of 0-10, optionally an integer in the range of 0-5, and n1 + m1 ≤ 10, n2 + m2 ≤ 10, and n3 + m3 ≤ 10.

In the present invention, all the descriptions about the fullerene derivatives are applicable to the fullerene derivatives in perovskite solar batteries.

In any of optional embodiments, the perovskite solar battery comprises a back electrode, a hole transport layer, a perovskite layer, an electron transport layer, optionally a passivation layer, optionally a buffer layer, and a conductive glass.

In any of embodiments, the perovskite solar battery described in the present application is an inverted perovskite solar battery.

The perovskite solar batteries can be divided into formal- and inverted-types according to the difference of light incident surface. The inverted perovskite solar battery is a perovskite solar battery that may sequentially comprise a back electrode, an electron transport layer, a perovskite layer, a hole transport layer, and a conductive glass.

The fullerene derivatives described in the present application are more suitable for inverted perovskite solar batteries.

In the perovskite solar battery described in the present application, the LUMO energy level of the electron transport layer is greater than or equal to that of the perovskite layer, and the difference between the LUMO energy levels of the electron transport layer and the perovskite layer is in the range of 0 to 0.2 eV, optionally in the range of 0 to 0.1 eV.

The open circuit voltage of perovskite solar batteries is mainly determined by the perovskite layer. However, some deep energy level defects in the perovskite layer and the matching between the energy levels of the transport layer and the perovskite may affect the open circuit voltage of the solar battery. If the LUMO energy level of the electron transport layer is higher than that of the perovskite layer, the electron extraction will be difficult, and the carriers will be accumulated at the interface between the perovskite layer and the electron transport layer, thereby affecting the photon-to-electron conversion efficiency of the solar battery. When the LUMO energy level of the electron transport layer is within 0.2 eV lower than that of the perovskite, an improved carrier extraction can be achieved, the energy level gap therebetween can be reduced, and the loss of the open circuit voltage can be reduced, while the final solar battery device will have an increased voltage and thus obtain a higher photon-to-electron conversion efficiency.

In any of embodiments, in the inverted perovskite solar battery described in the present application, no passivation layer is comprised between the perovskite layer and the electron transport layer.

According to the present application, a fullerene derivative useful for both the electron transport and the interface passivation described in the present application is obtained by modifying the fullerene. Using the fullerene derivative as the electron transport layer can passivate defects of the perovskite and stabilize the perovskite phase without introducing a passivation layer between the electron transport layer and the perovskite layer. In addition, the energy level of the fullerene derivative is changed by adjusting specific functional groups, and the resulting electron transport layer is more matched with the perovskite layer, thereby achieving a better photon-to-electron conversion efficiency. In addition, due to the omission of the passivation layer between the electron transport layer and the perovskite layer, the production cost is also saved and the production efficiency is improved.

### BRIEF DESCRIPTION OF THE DRAWINGS

FIG. 1 is a schematic diagram showing the structure of an inverted perovskite solar battery in an embodiment. The inverted perovskite solar battery sequentially comprises, from top to bottom, a glass, a fluorine-doped tin oxide film (FTO), a hole transport layer, a perovskite layer, an electron transport layer, a passivation layer/buffer layer, and a metal electrode, in which the glass and FTO form a conductive glass, and sunlight enters from the glass below.
FIG.2 is a graph showing the C NMR spectrum of the PCBM compound in Comparative Embodiment 2 of the present application.
FIG.3 is a graph showing the C NMR spectrum of the PCBM-1 compound in Embodiment 1 of the present application.
FIG.4 is a graph showing the C NMR spectrum of the PCBM-2 compound in Embodiment 2 of the present application.
FIG.5 is a graph showing the C NMR spectrum of the PCBM-3 compound in Embodiment 3 of the present application.
FIG.6 is a graph showing the C NMR spectrum of the PCBM-4 compound in Embodiment 4 of the present application.
FIG.7 is a graph showing the C NMR spectrum of the PCBM-5 compound in Embodiment 5 of the present application.
FIG.8 is a graph showing the C NMR spectrum of the PCBM-6 compound in Embodiment 6 of the present application.
FIG.9 is a graph showing the C NMR spectrum of the PCBM-7 compound in Embodiment 6 of the present application.

### DETAILED DESCRIPTION

Embodiments in which a fullerene derivative and a perovskite solar battery containing the same in the present application are specifically disclosed are described in detail below with reference to the accompanying drawings, as appropriate. However, there are cases where unnecessary detailed descriptions are omitted. For example, there are cases where detailed descriptions of well-known items and repeated descriptions of actually identical structures are omitted. This is to avoid unnecessary redundancy in the following descriptions and to facilitate the understanding by those skilled in the art. In addition, the drawings and subsequent descriptions are provided for those skilled in the art to fully understand the present application, and are not intended to limit the subject matter recited in the claims.

The "range" disclosed in the present application is defined in terms of lower and upper limits, and a given range is defined by selecting a lower limit and an upper limit, which define the boundaries of a particular range. A range defined in this manner may be inclusive or exclusive of end values, and may be arbitrarily combined, that is, any lower limit may be combined with any upper limit to form a range. For example, if ranges of 60-120 and 80-110 are listed for a particular parameter, it is understood that ranges of 60-110 and 80-120 are also expected. Additionally, if the minimum range values 1 and 2 are listed, and if the maximum range values 3, 4 and 5 are listed, the following ranges are all contemplated: 1-3, 1-4, 1-5, 2-3, 2-4 and 2-6. In the present application, unless stated otherwise, the numerical range "a-b" represents an abbreviated representation of any combination of real numbers between a and b, wherein both a and b are real numbers. For example, the numerical range "0-5" means that all real numbers between "0-5" have been listed herein, and "0-5" is just an abbreviated representation of the combination of these numerical values. Additionally, when it is stated that a certain parameter is an integer of ≥ 2, it is equivalent to disclosing that the parameter is, for example, an integer of 2, 3, 4, 5, 6, 7, 8, 9, 10, 11, 12, etc.

Unless otherwise specified, all embodiments and optional embodiments of the present application may be combined with each other to form new technical solutions.

Unless otherwise specified, all technical features and optional technical features of the present application can be combined with each other to form new technical solutions.

Unless otherwise specified, all steps of the present application may be performed sequentially or randomly, and optionally sequentially. For example, the method comprises steps (a) and (b), meaning that the method may comprise steps (a) and (b) performed sequentially, or may comprise steps (b) and (a) performed sequentially. For example, the reference to the method may further comprise step (c), meaning that step (c) may be added to the method in any order, for example, the method may comprise steps (a), (b) and (c), or may comprise steps (a), (c) and (b), or may comprise steps (c), (a) and (b), and so on.

Unless otherwise specified, the terms "include/including" and "comprise/comprising" mentioned in the present application may be open-ended or closed-ended. For example, the "including" and "comprising" may indicate that it is possible to include or comprise other components not listed, and it is also possible to include or comprise only the listed components.

Unless otherwise specified, the term "or" is inclusive in the present application. By way of example, the phrase "A or B" means "A, B, or both A and B". More specifically, the condition "A or B" is satisfied by any of the following: A is true (or present) and B is false (or absent); A is false (or absent) and B is true (or present); or both A and B are true (or present).

For perovskite solar batteries, the perovskite layer will have many defects during the preparation process, such as iodine vacancies, lead dangling bonds, etc., and the formed perovskite layer is unstable and easily change into other phases (for example, α phase into δ phase) due to distortion, thereby leading to a greatly reduced efficiency. In addition, the perovskite decomposes after exposure to moisture and/or oxygen, often resulting in the interface passivation of the perovskite layer. Interface passivation can eliminate many defects on perovskite and stabilize its perovskite phase. However, the introduction of an additional interface, while enabling the passivation of the perovskite layer, also leads to other problems, such as adverse effects on the conductivity of the passivated material, the presence of other interfacial problems, etc.

In addition, in the inverted perovskite solar batteries, the material used in the electron transport layer is mainly C60 fullerene at first. However, its low solubility is not favorable for the preparation of the electron transport layer. The emergence of PCBM ([6,6]-phenyl-C61-butyric acid methyl ester) solves this problem. Compared with C60 fullerene, the solubility of PCBM is greatly increased, and the solubility of PCBM is now sufficient for the thickness of the electron transport layer. However, in practice, the inventors of the present application have found that there are still other problems in the use of PCBM, such as energy level matching problems and interface problems. These problems, if not solved, may lead to the loss of carriers in the extraction process, resulting in poor solar battery performance.

Unexpectedly, the inventors of the present application have found that some specific modifications to fullerene materials, especially PCBM, may be able to better match the energy levels of the electron transport layer and the perovskite layer, stabilize the perovskite phase, and improve the interface problem in the perovskite without redundant interface introduction, thus improving both the stability and photon-to-electron conversion efficiency of the perovskite solar batteries.

Accordingly, a first aspect of the present application provides a fullerene derivative having a C60 fullerene group and a group of a compound of formula (1), formula (2) and/or formula (3) attached thereto, wherein the structural formulas of the compounds of formula (1), formula (2), and formula (3) are as follows: 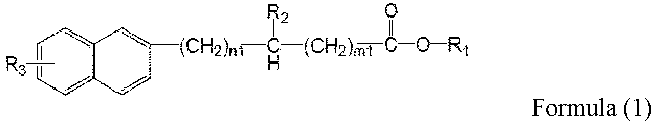 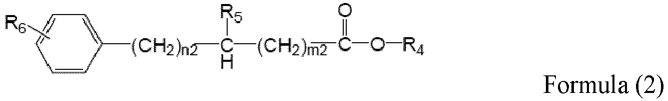 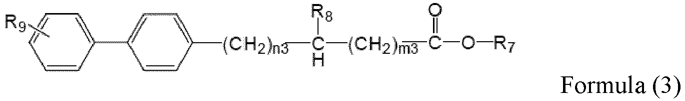 wherein
R₁, R₄, and R₇ are each independently selected from R', a phenyl group, a naphthyl group, a biphenyl group, R' substituted with a halogen, a phenyl group substituted with a halogen, a naphthyl group substituted with a halogen, a biphenyl group substituted with a halogen, a nitrogen-containing group, R' substituted with a nitrogen-containing group, a phenyl group substituted with a nitrogen-containing group, a naphthyl group substituted with a nitrogen-containing group or a biphenyl group substituted with a nitrogen-containing group;
R₂, R₅, and R₈ are each independently selected from hydrogen or R';
R₃, R₆, and R₉ are each independently selected from hydrogen, halogen, -O-R, a nitrogen-containing group, -OH, -OR, -NHCOR, -OCOR, -R, -CH₂COOH, a phenyl group or a naphthyl group;
the nitrogen-containing group is -N(R)₂, -NHR, -NH₂, a trimethylamine group, a triethylamine group or a tripropylamine group;
R' is an alkyl group with 1-5 carbon atoms;
R is an alkyl group with 1-10 carbon atoms;
n1, n2, n3, m1, m2, and m3 are each independently an integer in the range of 0-10, optionally an integer in the range of 0-5, and n1 + m1 ≤ 10, n2 + m2 ≤ 10, and n3 + m3 ≤ 10.

In the present application, the alkyl group with 1-5 carbon atoms can be a straight or branched alkyl group with 1-5 carbon atoms, which can be selected from, for example, methyl, ethyl, propyl, butyl, n-pentyl, isopropyl, isobutyl, tert-butyl, isopentyl, tert-pentyl or neopentyl. Optionally, the alkyl group with 1-5 carbon atoms is methyl.

In the present application, the alkyl group with 1-10 carbon atoms can be a straight or branched alkyl group with 1-10 carbon atoms, which can be selected from, for example, methyl, ethyl, propyl, butyl, pentyl, hexyl, heptyl, octyl, nonyl, decyl, isopropyl, isobutyl, tert-butyl, isopentyl, tert-pentyl, neopentyl, 2-methylpentyl, 3-methylpentyl, 2,2-dimethylbutyl, 2,3-dimethylbutyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, 2,2,3-trimethylbutyl, 2-methylheptyl, 3-methylheptyl, 4-methylheptyl, 2,2-dimethylhexane, 3,3-dimethylhexane, 2,3-dimethylhexane, 2,4-dimethylhexane, 2,5-dimethylhexane, 3,4-dimethylhexane, 3-ethylhexane, 2,2,3-trimethylpentane, 2,2,4-trimethylpentane, 2,3,3-trimethylpentane, 2,3,4-trimethylpentane, 2-methyl-3-ethylpentane, 3-methyl-3-ethylpentane, 2,2,3,3-tetramethylbutane, etc.

In the present application, halogen refers to fluorine, chlorine or bromine. Optionally, the halogen is fluorine.

The term "substituted with" described in this application, for example, described in "R' substituted with a halogen, a phenyl group substituted with a halogen, a naphthyl group substituted with a halogen, R' substituted with a nitrogen-containing group, a phenyl group substituted with a nitrogen-containing group, or a naphthyl group substituted with a nitrogen-containing group" can refer to a mono-substituted, di-substituted or multi-substituted group. For example, R' substituted with a halogen can be R' substituted with one, two or more halogens, a naphthyl group substituted with a halogen can be a naphthyl group substituted with one, two or more halogens, R' substituted with a nitrogen-containing group can be R' substituted with one, two, or more nitrogen-containing groups, a phenyl group substituted with a nitrogen-containing group can be a phenyl group substituted with one, two, or more nitrogen-containing groups, and a naphthyl group substituted with a nitrogen-containing group can be a naphthyl group substituted with one, two, or more nitrogen-containing groups.

In the present application, there is no limitation on the position of the attachment in groups of compounds of formula (1), formula (2), and formula (3) on the C60 fullerene group, as long as the attachment can be allowed. Theoretically, the position having a double bond in the C60 fullerene group can be attached to the groups of compounds of formula (1), formula (2), and formula (3).

In the present application, the position of the attachment to the C60 fullerene group in the compounds of formula (1), formula (2) and formula (3) is determined by the reaction mechanism. Optionally, the groups of compounds of formula (1), formula (2), and formula (3) can also be attached to the C60 fullerene group at positions (CHz)m or (CHR₂), (CH₂)ₙ₂ or (CHR₅), and (CH₂)ₙ₃ or (CHR₈). Optionally, the compound of formula (1), formula (2) or formula (3) is attached to the C60 fullerene group at the first aliphatic carbon atom adjacent to the benzene or naphthalene ring in the compound of formula (1), formula (2) or formula (3).

The fullerene derivatives obtained by introducing one or more specific functional groups (e.g., halogens, nitrogen-containing groups, aryl groups, etc.) to PCBM can stabilize the phase stability of the perovskite and passivate the interfacial defects, thereby improving the photon-to-electron conversion efficiency and stability of the perovskite solar battery. Without being bound by any theory, it is speculated that the stabilizing effect of perovskite solar batteries and the improvement of the photon-to-electron conversion efficiency of the batteries by these functional groups may be attributed to the fact that these introduced functional groups can increase the binding energy to the proton at position A in perovskite (chemical formula: ABX₃) and can be better anchored with lead, if present, thereby increasing the phase stability of the perovskite. In addition, they can reduce the non-radiative recombination, so that the interfacial defects of the perovskite can be passivated. In addition, different functional groups have different effects when introduced into different positions. For example, the introduction of certain electron-withdrawing functional groups (such as halogens) can passivate interfacial defects on the perovskite surface, and the introduction of certain electron-donating functional groups (such as nitrogen-containing groups and aryl groups (for example phenyl group, naphthyl group)) can adjust the LUMO energy level and HOMO energy level of perovskite.

Thus, the fullerene derivatives described in the present application can improve the photon-to-electron conversion efficiency and stability of perovskite solar batteries.

In addition, the introduction of hydrophobic functional groups (a alkyl group, etc.) can prevent the decomposition of perovskite due to water absorption.

In some embodiments, in formula (1) and formula (2),
R₁ is selected from R';
R₂ and R₅ are hydrogen;
R₃ is hydrogen;
R₄ is selected from R', a phenyl group or a trimethylamine group;
R₆ is selected from hydrogen or halogen, optionally hydrogen or fluorine;
n1 + m1 ≤ 5; and
n2 + m2 ≤ 5.

Optionally, the sum of n1 and m1 may be 3; and the sum of n2 and m2 may be 3.

In some embodiments, in formula (1), formula (2), and formula (3), each C60 fullerene group is attached to 1-3, optionally 2, groups selected from the groups of compounds of formula (1), formula (2) and/or formula (3).

The number of the groups of a compound of formula (1), formula (2) and/or formula (3) attached to the C60 fullerene group may affect the energy level position of the fullerene. As this number increases, the LUMO energy level decreases. However, if this number increases further, the steric hindrance will be increased, which is not conducive to the accumulation of fullerene derivatives, and will reduce the electron transport ability, thereby reducing long-term stability of perovskite solar batteries. Therefore, each C60 fullerene group is optionally attached to 1-3, optionally 2, groups selected from the groups of compounds of formula (1), formula (2) and/or formula (3).

The LUMO energy level of the fullerene derivatives according to the present application ranges from -4.02 to -3.72 eV, optionally from -4.02 to -3.82 eV, and more optionally from -4 to -3.92 eV.

Although the HOMO and LUMO energy levels of the fullerene derivatives are mainly provided by C60, the introduction of the above-mentioned specific functional groups will reduce the LUMO energy levels of the fullerene derivatives described in the present application to better match the energy levels of the conduction band minimum of the perovskite. Thus, the electrons are more easily separated at the interface between the electron transport layer and the perovskite layer made of the fullerene derivatives, reducing the recombination of carriers at the interface, and thus improving the photon-to-electron conversion efficiency of the perovskite solar batteries.

In some embodiments, the fullerene derivative can have the structure of formula (4), formula (5), formula (6), formula (7), formula (8), formula (9), formula (10), formula (11) or formula (12): 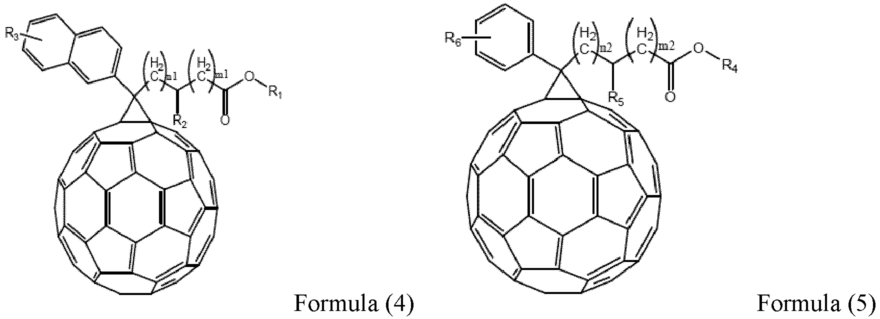 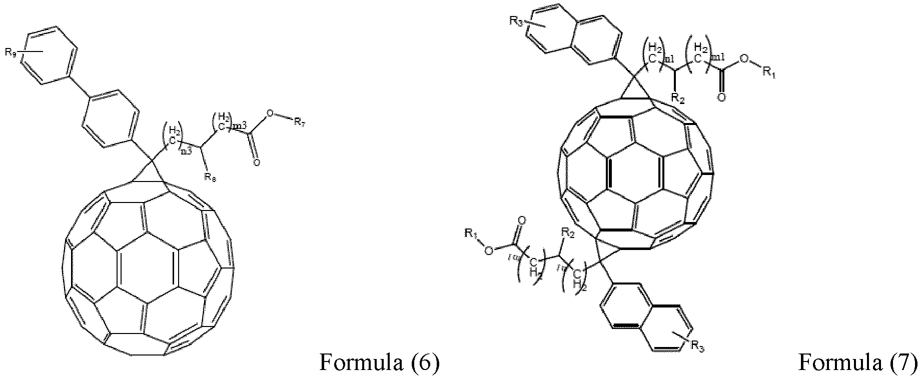 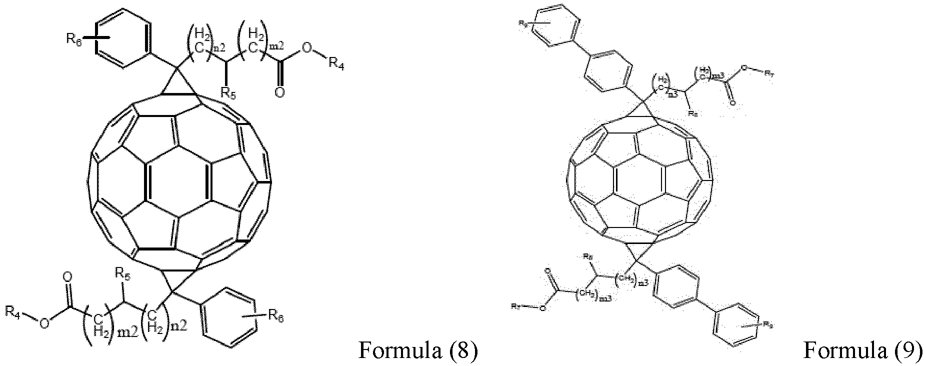 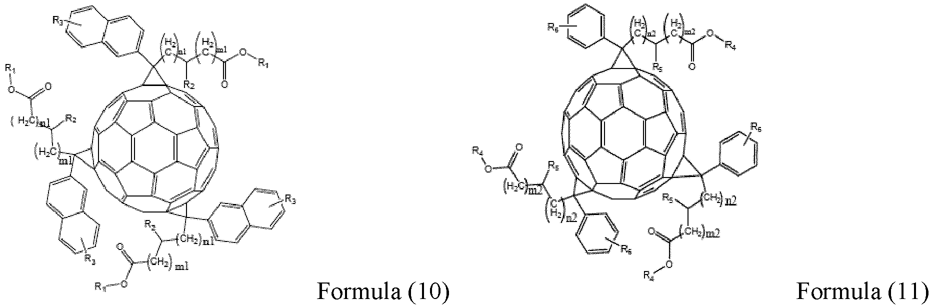 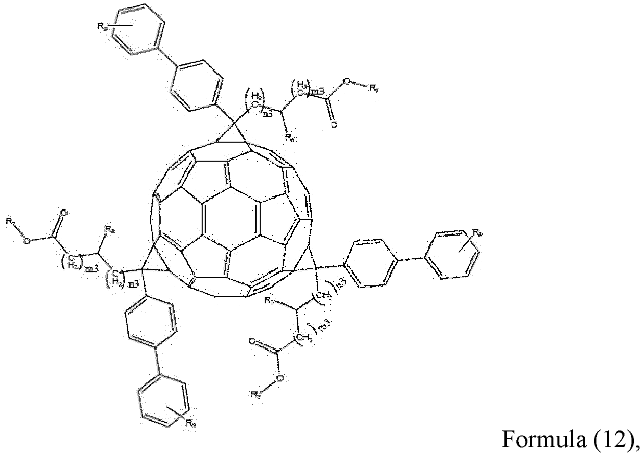 wherein R₁ to R₉, n1 to n3, and m1 to m3 are as defined in formulas (1), (2) and (3).

Among the above structural formulas, formula (4), formula (7), and formula (10) are those in which one, two, and three compounds of general formula (1) are respectively attached to C60 fullerene; formula (5), formula (8), and formula (11) are those in which one, two, and three compounds of formula (2) are respectively attached to C60 fullerene; and formula (6), formula (9), and formula (12) are those in which one, two, and three compounds of formula (3) are respectively attached to C60 fullerene.

It should be understood that the specific attachment positions of the compounds of formula (1), formula (2), and formula (3) to fullerene as drawn in formula (4), formula (5), formula (6), formula (7), formula (8), formula (9), formula (10), formula (11) or formula (12) are intended to facilitate a more intuitive understanding of the present application and are not intended to limit the specific attachment positions. In the present application, compounds of formula (1), formula (2), and formula (3) can be attached to C60 fullerene at any double bond position, and one or more compounds of formula (1), formula (2), and formula (3) can also be attached to C60 fullerene, as long as the attachment is chemically feasible. Optionally, the compounds of formula (1), formula (2), and formula (3) are attached to C60 fullerene groups via their carbon atoms adjacent to the benzene or naphthalene ring, respectively.

The fullerene derivatives described in the present application can be prepared by methods similar to those used for the preparation of PCBM, for example, by methods similar to those described in the research paper "Synthesis and purification process of PCBM" documented in the Journal of Chemical Engineering, Vol. 66, No. S2 (Aug. 2015).

Alternatively, the method for preparing the fullerene derivatives described in the present application may comprise the following steps of:
(1) obtaining the precursor 1 of the formula (1), (2) or (3) using a synthetic method known in the art or through commercial purchase, the precursor 1 being similar in structure to formula (1), (2) or (3), differing only in that the aliphatic carbon connected to the benzene or naphthalene ring in the precursor 1 is double-bonded to an oxygen atom to form a carbonyl group;
(2) reacting the precursor 1 with a compound containing sulfonyl hydrazide (optionally p-toluenesulfonyl hydrazide) to synthesize the precursor 2, the precursor 2 being similar in structure to formula (1), (2) or (3), differing only in that the aliphatic carbon connected to the benzene or naphthalene ring in the precursor 2 is double-bonded with a sulfonyl hydrazone (optionally benzenesulfonyl hydrazone); optionally, the reaction comprising the following steps of: dissolving the precursor 1 and the compound containing sulfonyl hydrazide (optionally p-toluenesulfonyl hydrazide) in a solvent, heating to reflux for 2-15 h, then cooling, subsequently storing in the dark for 6-20 h, and then placing at -5°C to -20°C for 12-48 h to obtain the precursor 2; and
(3) reacting the precursor 2 with C60 fullerene to obtain the fullerene derivative described in the present application; optionally, the reaction comprising the following steps of:
   A. mixing the precursor 2 with sodium methoxide and then dissolving in pyridine, withdrawing the air from the reaction system and charging it with an inert gas (such as nitrogen) so as to remove most of the moisture and oxygen in the reactor chamber and the reaction system;
   B. dissolving the C60 fullerene in a solvent (such as o-dichlorobenzene), then adding the obtained C60 fullerene solution dropwise to the above reaction system, and allowing the reaction system to react at 50-90°C for 12-32 h after the dropwise addition; after completing the reaction, removing the solvent, and applying the product as obtained directly to the next step without purification; and
   C. dissolving the product obtained in step B in a solvent (such as o-dichlorobenzene), reacting at 150-210°C for 14-34 h, then removing most of the solvent, and purifying through a silica gel chromatography column using toluene as the mobile phase to obtain the fullerene derivatives described in the present application.

A second aspect of the present application provides a perovskite solar battery, which comprises a conductive glass, a hole transport layer, a perovskite layer, an electron transport layer comprising a fullerene derivative and a back electrode, the fullerene derivative having a C60 fullerene group and a group of a compound of formula (1), formula (2) and/or formula (3) attached thereto, wherein the structural formulas of the compounds of formula (1), formula (2), and formula (3) are as follows: 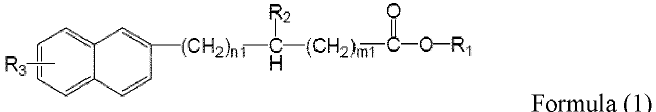 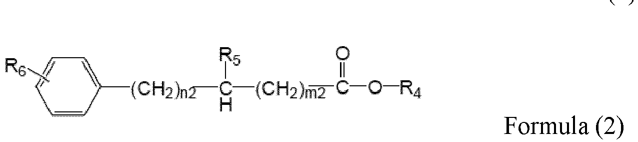 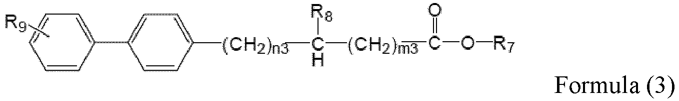 wherein
R₁, R₄, and R₇ are each independently selected from R', a phenyl group, a naphthyl group, a biphenyl group, R' substituted with a halogen, a phenyl group substituted with a halogen, a naphthyl group substituted with a halogen, a biphenyl group substituted with a halogen, a nitrogen-containing group, R' substituted with a nitrogen-containing group, a phenyl group substituted with a nitrogen-containing group, a naphthyl group substituted with a nitrogen-containing group or a biphenyl group substituted with a nitrogen-containing group;
R₂, R₅, and R₈ are each independently selected from hydrogen or R';
R₃, R₆, and R₉ are each independently selected from hydrogen, halogen, -O-R, a nitrogen-containing group, -OH, -OR, -NHCOR, -OCOR, -R, -CH₂COOH, a phenyl group or a naphthyl group;
the nitrogen-containing group is -N(R)₂, -NHR, -NH₂, a trimethylamine group, a triethylamine group or a tripropylamine group;
R' is an alkyl group with 1-5 carbon atoms;
R is an alkyl group with 1-10 carbon atoms;
n1, n2, n3, m1, m2, and m3 are each independently an integer in the range of 0-10, optionally an integer in the range of 0-5, and n1 + m1 ≤ 10, n2 + m2 ≤ 10, and n3 + m3 ≤ 10.

In some optional embodiments, in the perovskite solar battery, the fullerene derivative used in the electron transport layer has a structure of formula (4), formula (5), formula (6), formula (7 ), formula (8), formula (9), formula (10), formula (11) or formula (12).

In some optional embodiments, the perovskite solar battery comprises a back electrode, a hole transport layer, a perovskite layer, an electron transport layer, optionally a passivation layer, optionally a buffer layer, and a conductive glass.

The structural components of the perovskite solar battery described in the present application are introduced below, but the present application is not limited thereto.

### Back electrode

The back electrode can be any electrode used in the art. Optionally, the back electrode is a metal electrode. The metal electrode can be made of gold (Au), silver (Ag), or copper (Cu), but not limited thereto. The metal electrode can be prepared by vapor deposition.

The thickness of the back electrode can be any thickness used in the art. Optionally, the thickness of the back electrode is 10-200 nm. The back electrode should not be too thick, otherwise it will fall off easily.

### Electron transport layer

The electron transport layer is prepared using the fullerene derivative described in the first aspect of the present application. The electron transport layer may comprise the fullerene derivatives described in the present application alone. Optionally, in addition to the fullerene derivative, the electron transport layer may also comprise other materials that can be used for the electron transport layer of the perovskite solar battery.

The electron transport layer can be prepared by conventional methods in the art, and can also be prepared by the following method. The fullerene derivative is dissolved in an organic solvent (such as chlorobenzene, dichlorobenzene, toluene, or xylene) to prepare a solution of fullerene derivative in the concentration range of 5-50 mg/ml. Then it is covered on the surface of the passivation layer or the perovskite layer (if there is no passivation layer between the perovskite layer and the electron transport layer). The covering can be carried out by a spin-coating process via a spin coater, in which the rotational speed can be 500-5000 rpm and the spin-coating time can be 5-50 s. After spin-coating, annealing is carried out, and the annealing temperature can be 80-150°C and the annealing time can be 5-60 min. The electron transport layer is obtained after annealing.

The thickness of the electron transport layer can be any thickness used in the art. Optionally, the thickness of the electron transport layer is 30-120 nm.

### Passivation layer/buffer layer

Typically, a passivation layer/buffer layer is used between the electron transport layer and the back electrode. For example, bathocuproine is used as a buffer layer to enhance the performance of solar batteries.

### Perovskite layer

The chemical formula of the perovskite light-absorbing layer satisfies ABX₃, in which A is methylamine (MA for short), formamidine (FA for short) or cesium (Cs), B is lead (Pb) or tin (Sn), and X is iodine (I) or bromine (Br).

Optionally, a formamidinium lead iodide (FAPbI₃) system can be used as a material in the perovskite layer. The LUMO energy level of the FAPbI₃ system is optionally -4.02±0.3 eV, more optionally -4.02±0.2 eV, even more optionally -4.02±0.15 eV.

The perovskite layer can be prepared by conventional technical means in the art, and can also be prepared by the following method. Taking the inverted perovskite solar battery as an example, the perovskite precursor material, such as formamidine iodide (FAI), lead iodide (PbI₂), methylamine chloride (MACl), methylammonium iodide (MAI), cesium iodide (CsI), etc., is weighed, dissolved in a solvent (for example, dimethylformamide (DMF), dimethyl sulfoxide (DMSO), etc.), stirred evenly, and filtered to remove the supernatant. The supernatant is covered on the prepared hole transport layer, in which the covering can be carried out by a spin-coating process via a spin coater, the rotational speed can be 500-5000 rpm and the spin-coating time can be 5-50 s. After covering, annealing is carried out, in which the annealing temperature can be 80-150°C and the annealing time can be 0-60 min. The perovskite layer is obtained after annealing.

The thickness of the perovskite layer can be any thickness used in the art. Optionally, the thickness of the perovskite layer is 200-1000 nm.

### Hole transport layer

The hole transport layer is used to collect and extract holes from the perovskite layer. The material in the hole transport layer may be a material conventionally used in the art, such as nickel oxide (NiOx), poly(triarylamine) (PTAA), and the like. The hole transport layer can be prepared by conventional technical means in the art. For example, the nickel oxide hole transport layer can be prepared by the following method. Nickel nitrate, nickel acetylacetonate or nickel acetate is dissolved in methanol, and covered on the cleaned conductive glass. The covering can be carried out by a spin-coating process via a spin coater, the rotational speed can be 500-5000 rpm and the spin-coating time can be 5-50 s. After spin-coating, annealing is carried out, in which the annealing temperature can be 80-400°C and the annealing time can be 0-120 min. The hole transport layer is obtained after annealing.

The thickness of the hole transport layer can be any thickness used in the art. Optionally, the thickness of the hole transport layer is 10-100 nm.

### Conductive glass

The conductive glass usually has a certain degree of transparency. Generally, a transparent conductive glass is used. The conductive glass usually consists of a glass substrate and an oxide thin film (TCO for short) conductive layer. Commonly used TCOs include indium tin oxide (ITO) and fluorine-doped tin oxide (FTO), but the present application is not limited thereto. The conductive glass is generally any conductive glass used in the art. The conductive glass is commercially available.

Before use, the conductive glass needs to be washed, for example, by ultrasonic cleaning with detergent, deionized water, ethanol and the like.

In an optional embodiment, there is a passivation layer between the hole transport layer and the perovskite layer for passivating defects at the interface between the hole transport layer and the perovskite layer.

Optionally, there is a passivation layer between the electron transport layer and the perovskite layer for passivating defects at the interface between the electron transport layer and the perovskite layer.

In some embodiments, the perovskite solar battery described in the present application is an inverted perovskite solar battery.

The perovskite solar batteries can be divided into formal- and inverted-types according to the difference of light incident surface. The inverted perovskite solar battery is a perovskite solar battery that may sequentially comprise a back electrode, an electron transport layer, a perovskite layer, a hole transport layer, and a conductive glass.

In some optional embodiments, the perovskite solar battery sequentially comprises a back electrode, optionally a passivation layer/buffer layer, an electron transport layer, a perovskite layer, optionally a passivation layer, a hole transport layer, and a conductive glass.

The fullerene derivatives described in the present application are more suitable for inverted perovskite solar batteries.

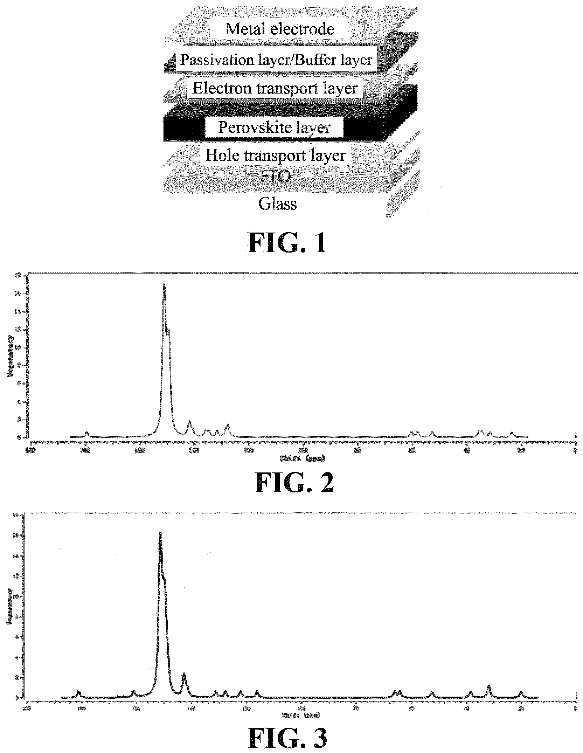

FIG. 1 is a schematic diagram showing the structure of an inverted perovskite solar battery in one embodiment of the present application. The inverted perovskite solar battery sequentially comprises, from top to bottom, a glass, a fluorine-doped tin oxide film (FTO), a hole transport layer, a perovskite layer, an electron transport layer, a passivation layer/buffer layer, and a metal electrode, in which the glass and FTO form a conductive glass, and sunlight enters from the glass below.

In the perovskite solar battery described in the present application, the LUMO energy level of the electron transport layer is greater than or equal to that of the perovskite layer, and the difference between the LUMO energy levels of the electron transport layer and the perovskite layer is in the range of 0 to 0.2 eV, optionally in the range of 0 to 0.1 eV.

In some optional embodiments, the LUMO energy levels of the electron transport layer and the perovskite layer are not equal, and the difference between the LUMO energy levels of the electron transport layer and the perovskite layer is in the range of 0.07-0.13 eV, optionally about 0.1 eV.

The open circuit voltage of perovskite solar batteries is mainly determined by the perovskite layer. However, some deep energy level defects in the perovskite layer and the matching between the energy levels of the transport layer and the perovskite may affect the open circuit voltage of the solar battery. If the LUMO energy level of the electron transport layer is higher than that of the perovskite layer, the electron extraction will be difficult, and the carriers will be accumulated at the interface between the perovskite layer and the electron transport layer, thereby affecting the photon-to-electron conversion efficiency of the solar battery. When the LUMO energy level of the electron transport layer is within 0.2 eV lower than that of the perovskite, an improved carrier extraction can be achieved, the energy level gap therebetween can be reduced, and the loss of the open circuit voltage can be reduced, while the final solar battery device will have an increased voltage and thus obtain a higher photon-to-electron conversion efficiency.

In some embodiments, the inverted perovskite solar battery described in the present application does not comprise a passivation layer between the perovskite layer and the electron transport layer.

Optionally, no other layers are comprised between the perovskite layer and the electron transport layer.

According to the present application, a fullerene derivative useful for both the electron transport and the interface passivation described in the present application is obtained by modifying the fullerene. Using the fullerene derivative as the electron transport layer can passivate defects of the perovskite and stabilize the perovskite phase without introducing a passivation layer between the electron transport layer and the perovskite layer. In addition, the energy level of the fullerene derivative is changed by adjusting specific functional groups, and the resulting electron transport layer is more matched with the perovskite layer, thereby achieving a better photon-to-electron conversion efficiency. In addition, due to the omission of the passivation layer between the electron transport layer and the perovskite layer, the production cost is also saved and the production efficiency is improved.

### EMBODIMENTS

Embodiments of the present application will be described hereinafter. The embodiments described below are exemplary and only used to explain the present application, and are not to be construed as limiting the present application. Where specific techniques or conditions are not specified in the embodiments, the techniques or conditions described in the literatures of the art or the product specifications are followed. All of the used agents or instruments which are not specified with the manufacturer are conventional and commercially available products.

The material and source used in the embodiment are as follows: 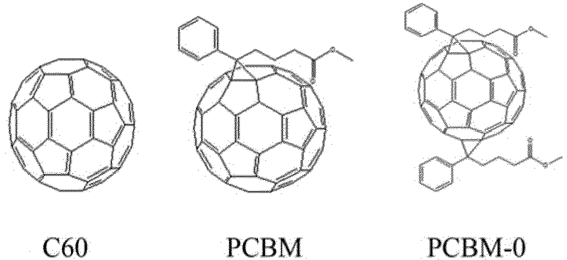 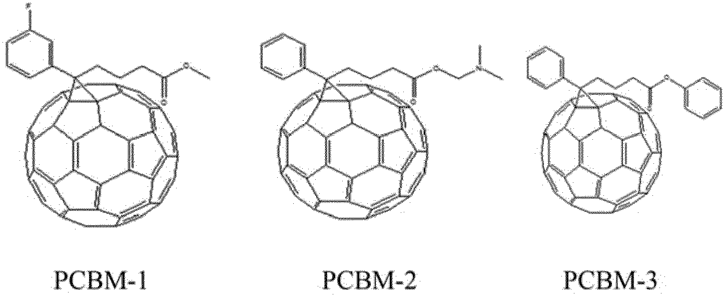 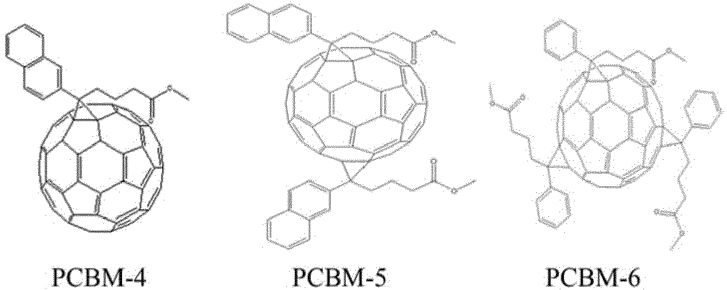 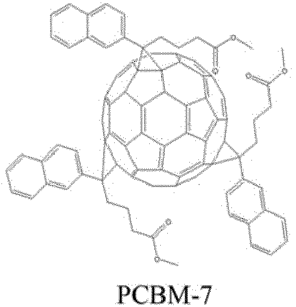
C60 fullerene: CAS No. 99685-96-8, molecular weight 720.64, commercially available
PCBM: [6,6]-phenyl-C61-butyric acid methyl ester, CAS No. 160848-22-6, molecular weight 910.88, commercially available
PCBM-0: [6,6]-diphenyl-C62 bis (butyric acid methyl ester), commercially available from Sigma-Aldrich
PCBM-1: [6,6]-fluorophenyl-C61-butyric acid methyl ester; see Embodiment 1 for the preparation method
PCBM-2: [6,6]-phenyl-C61-butyric acid dimethylaminomethyl ester; see Embodiment 2 for the preparation method
PCBM-3: [6,6]-phenyl-C61-butyric acid phenyl ester; see Embodiment 3 for the preparation method
PCBM-4: [6,6]-naphthyl-C61-butyric acid methyl ester; see Embodiment 4 for the preparation method
PCBM-5: [6,6]-dinaphthyl-C62-bis (butyric acid methyl ester); see Embodiment 5 for the preparation method
PCBM-6: [6,6]-triphenyl-C63-tris (butyric acid methyl ester); see Embodiment 6 for the preparation method
PCBM-7: [6,6]-trinaphthyl-C63-tris (butyric acid methyl ester); see Embodiment 7 for the preparation method

### Embodiment 4

### Step 1: Preparation of PCBM-4

S1: Synthesis of glutaric acid monomethyl ester
25 g of glutaric anhydride was dispersed in 12 ml of methanol and heated to reflux for 12 h. Then the reaction was stopped, and excess methanol was removed by a rotary evaporator to obtain glutaric acid monomethyl ester, which was directly used in the next step without purification.

### S2: Synthesis of acyl chloride (4-chloroformylbutyric acid methyl ester)

The glutaric acid monomethyl ester obtained in step S1 was evacuated and then charged with nitrogen, so that the reaction was placed under a nitrogen (N₂) atmosphere to isolate moisture and oxygen. Thionyl chloride was added, and a tail gas treatment device was connected. The molar ratio of glutaric acid monomethyl ester to thionyl chloride is 1:1.3. The reaction was stirred until no air bubbles were generated. After that, the reaction was heated to reflux and continued until no air bubbles are generated. The reaction was then stopped and excess sulfoxide chloride was removed by a rotary evaporator to give the acyl chloride product, which was used directly in the next step without purification.

### S3: Synthesis of methyl naphthylformylbutyrate

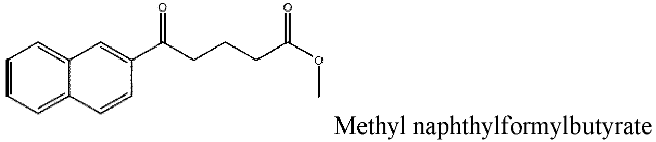

34 g of anhydrous aluminum chloride was ground, dispersed in 200 ml of naphthalene, evacuated and then charged with nitrogen, so that the reaction was placed under a nitrogen atmosphere to isolate moisture and oxygen. Then, the reaction was stirred in an ice-water bath for half an hour, 35 g of the acid chloride product obtained in step 2 was slowly added dropwise, and allowed to react overnight after the dropwise addition. After the reaction was completed, the reaction product was poured into a mixture of hydrochloric acid and ice to completely disperse the product into the solution. Methyl naphthylformylbutyrate in the product was extracted with ethyl acetate. The organic extracts were mixed after several extractions to obtain the organic phase solution of S3 product, which was concentrated to a constant weight by rotary evaporation using a rotary evaporator to obtain the Methyl naphthylformylbutyrate product. This product was used directly in the next step without purification.

### S4: Synthesis of methyl naphthylformylbutyrate benzenesulfonyl hydrazone

20 g of methyl naphthylformylbutyrate obtained in step S3 and 15 g of p-toluenesulfonylhydrazide were dissolved in 200 ml of methanol, heated to reflux for 6 h, then cooled to room temperature, stored in the dark for 12 hours, then transferred to a refrigerator, and stored at -15°C for 24h to obtain the product hydrazone. The product hydrazone was filtered by suction, rinsed with methanol, and then oven dried to obtain a pure methyl naphthylformylbutyrate benzenesulfonyl hydrazone with a purity of 99.5%.

### S5: Synthesis of PCBM-4

5 g of methyl naphthylformylbutyrate benzenesulfonyl hydrazone obtained in step S4 and 1 g of sodium methoxide were accurately weighed and placed in a flask, and dissolved in 100 ml of pyridine upon mixing. The reaction system was evacuated and charged with nitrogen so as to remove most of the moisture and oxygen in the reactor chamber and the reaction system.

4 g of C60 fullerene was dissolved in 200 ml of o-dichlorobenzene, and then the solution of the obtained C60 fullerene in o-dichlorobenzene was added dropwise to the above system. The reaction system was allowed to react at 75°C for 22 h after the dropwise addition. After the reaction was completed, the solvent was removed using a rotary evaporator and the resulting product was used directly in the next step without purification.

2 g of the product obtained in the previous step was dissolved in 100 ml of o-dichlorobenzene and allowed to react at 180°C for 24 h. After that, most of the solvent was removed using a rotary evaporator. The column separation was carried out through a silica gel chromatography column using toluene as a mobile phase, and the eluates were C60, PCBM-4, and other by-products, respectively. The eluate of PCBM-4 in toluene was the desired material. The eluate of PCBM-4 in toluene was concentrated using a rotary evaporator to remove most of the solvent, then methanol was added to precipitate the product, and then centrifuged and oven dried to obtain the target product PCBM-4.

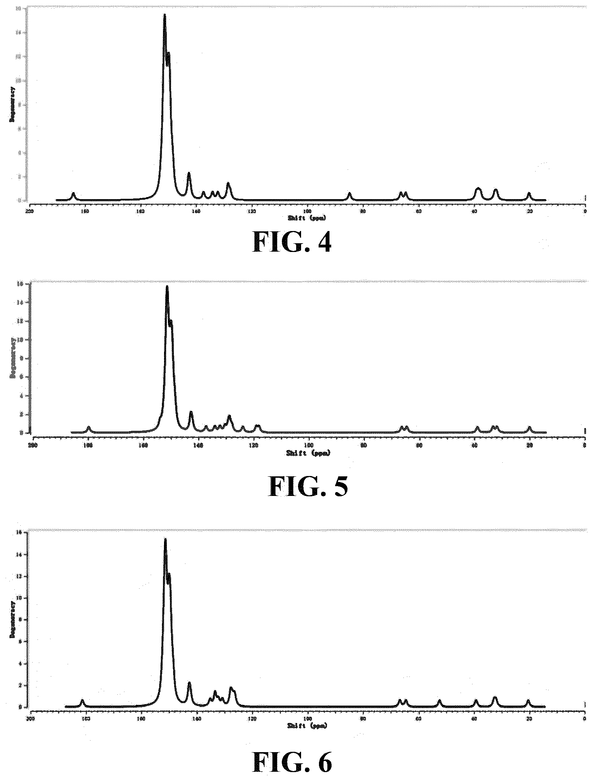

See FIG.6 for the C NMR spectrum of PCBM-4.

### Step 2: Preparation of perovskite solar battery

### [Conductive Glass]

The conductive glass with a fluorine-doped tin oxide (FTO) oxide film was commercially available and used directly after cleaning.

### [Hole transport layer]

48.3 mg of nickel nitrate hexahydrate was weighed and dissolved in 1 ml of methanol. The reaction was stirred via a magnetic stirrer for 2 h to obtain a light green transparent liquid. The liquid was filtered to remove the supernatant, which was spin-coated on a conductive glass, and spin-coating time was 30 s. Then annealing was performed according to the following procedure: holding at 80°C for 10 min, ramping up to 300°C within half an hour, then holding at 300°C for 45 min, then cooling to 100°C and removing to obtain a battery assembly sequentially comprising a conductive glass and a hole transport layer.

### [Passivation layer]

0.5 mg of poly[bis(4-phenyl) (2,4,6-trimethylphenyl)amine] (PTAA) was dissolved in 1 mL of chlorobenzene upon stirring, then 60 µL was removed to cover on the surface of nickel oxide, spun for 30 s at 5000 rpm, removed, and annealed by heating at 100°C for 10 min to obtain the passivated layer.

### [Perovskite layer]

Preparation of the perovskite solution: 80 mg of formamidine iodide (FAI), 223 mg of lead iodide (PbI₂), and 15 mg of methylamine chloride (MACl) were dissolved in 1 mL of solvent, in which said solvent was a mixed solvent of N,N-dimethylformamide (DMF) and dimethyl sulfoxide (DMSO), and the volume ratio of DMF to DMSO was 4:1 (DMF:DMSO). The perovskite solution was stirred for 1 h at room temperature using a magnetic stirrer, and filtered to remove the supernatant for further use.

Formamidine iodide (FAI), lead iodide (PbI₂), and methylamine chloride (MACl) were purchased from Xi'an p-OLED, and DMF and DMSO were purchased from Sigma.

60 µL of the supernatant of the perovskite solution was added dropwise on the hole transport layer, and spun for 15 s with a homogenizer. 600 µL of the anti-solvent chlorobenzene was added, and then annealed at 150°C for1 h to obtain a battery assembly sequentially comprising, from top to bottom, a conductive glass, a hole transport layer and a perovskite layer.

### [Electron transport layer and buffer layer]

Preparation of fullerene derivative solution: the solute was PCBM-4 prepared in step 1, the solvent was chlorobenzene, and the concentration was 20 mg/ml.

Preparation of bathocuproine (BCP, commercially available) solution: the concentration was 0.5 mg/mL, and the solvent is isopropanol;
60 µL of PCBM solution as prepared was spin-coated on the perovskite layer with a Leibo homogenizer, and the spin-coating time was 30 s. After that, it was annealed at 100°C for 10 min, then removed from the instrument and cooled to room temperature to obtain an electron transport layer. 60 µL of BCP solution was then spin-coated on the surface of the electron transport layer, and the spin-coating time was 30 s. After spin-coating, a buffer layer was obtained.

### [Metal electrode]

The battery assembly sequentially comprising a conductive glass, a hole transport layer, a passivation layer, a perovskite layer, an electron transport layer, and a buffer layer prepared in the previous step was scraped with a blade according to the mask pattern to remove some functional layers (including the hole transport layer, passivation layer, chalcogenide layer, electron transport layer, buffer layer) so as to expose the conductive glass layer. After which, the residual functional layer was wiped off with washing solution, and the assembly was placed in a vapor deposition mask. 80 nm silver was vapor-deposited on the exposed conductive glass in a vacuum vapor deposition equipment with a vapor deposition rate of 0.1 A/s. A complete perovskite solar battery was obtained after the vapor deposition was completed.

### Embodiment 1

The perovskite solar battery of Embodiment 1 was prepared in a manner similar to Embodiment 4, except that the naphthalene in step S3 was replaced with fluorobenzene, that is, methyl fluorophenylformylbutyrate was synthesized in step 1 instead of methyl naphthylformylbutyrate.

See FIG.3 for the C NMR spectrum of PCBM-1.

### Embodiment 2

The perovskite solar battery of Embodiment 2 was prepared in a manner similar to Embodiment 4, except that glutaric acid trimethylamine ester was synthesized in step S1, and naphthalene in step S3 was replaced with benzene, that is, PCBM-2 was prepared in step 1.

See FIG.4 for the C NMR spectrum of PCBM-2.

### Embodiment 3

The perovskite solar battery of Embodiment 3 was prepared in a manner similar to Embodiment 4, except that phenyl glutarate was synthesized in step S1, and naphthalene in step S3 was replaced with benzene, that is, PCBM-3 was prepared in step 1.

See FIG.5 for the C NMR spectrum of PCBM-3.

### Embodiment 5

The perovskite solar battery of Embodiment 5 was prepared in a manner similar to Embodiment 4, except that 10 g of methyl naphthylformylbutyrate benzenesulfonyl hydrazone obtained in Step S4 was used in Step S5, that is, step PCBM-5 was prepared in step 1.

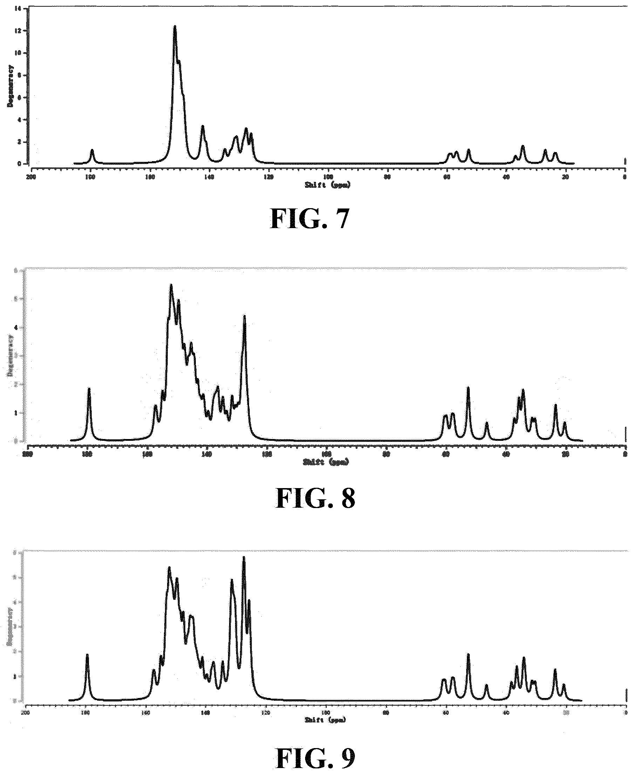

See FIG.7 for the C NMR spectrum of PCBM-5.

### Embodiment 6

The perovskite solar battery of Embodiment 6 was prepared in a manner similar to Embodiment 4, except that the corresponding benzene-containing compound was used instead of the naphthalene-containing compound and 15 g of methyl phenylformylbutyrate benzenesulfonyl hydrazone obtained in Step S4 was used in Step S5, that is, step PCBM-6 was prepared in step 1.

See FIG.8 for the C NMR spectrum of PCBM-6.

### Embodiment 7

The perovskite solar battery of Embodiment 7 was prepared in a manner similar to Embodiment 4, except that 15 g of methyl naphthylformylbutyrate benzenesulfonyl hydrazone obtained in Step S4 was used in Step S5, that is, step PCBM-7 was prepared in step 1.

See FIG.9 for the C NMR spectrum of PCBM-7.

The C NMR spectrum of PCBM-1 to PCBM-7 prepared in Embodiments 1-7 and the C NMR spectrum of PCBM are shown in FIG.3-9.

### Comparative Embodiment 1

The perovskite solar battery of Comparative Embodiment 1 was prepared in a manner similar to Embodiment 4, except that C60 fullerene was used instead of PCBM-4 in the electron transport layer.

### Comparative Embodiment 2

The perovskite solar battery of Comparative Embodiment 2 was prepared in a manner similar to Embodiment 4, except that PCBM was used instead of PCBM-4 in the electron transport layer.

### Comparative Embodiment 3

The perovskite solar battery of Comparative Embodiment 3 was prepared in a manner similar to Embodiment 4, except that PCBM-0 was used instead of PCBM-4 in the electron transport layer.

### Comparative Embodiment 4

The perovskite solar battery of Comparative Embodiment 4 was prepared in a manner similar to Comparative Embodiment 2, except that a passivation layer was added between the electron transport layer and the perovskite layer. The specific preparation method was as follows:

### [Passivation layer between the perovskite layer and the electron transport layer]

Preparation of passivation material solution: 0.5 mg of poly[bis(4-phenyl) (2,4,6-trimethylphenyl) amine] (PTAA) was dissolved in 1 ml of chlorobenzene to obtain a solution of PTAA in chlorobenzene, which was dissolved upon stirring.

60 µL of the prepared passivation material solution was covered on the surface of the perovskite layer, removed after rotating at 5000 rpm for 30 s, and annealed by heating at 100°C for 10 min to obtain a passivation layer.

### [Electron transport layer]

The electron transport layer was prepared in the same manner as in Comparative Embodiment 2, except that 60 µL of the prepared PCBM solution was spin-coated on the passivation layer obtained in the previous step.

### Performance Test

### 1. Method for testing photon-to-electron conversion efficiency (I-V)

The test was carried out according to the national standard IEC61215, in which the test was carried out using a digital source meter in the presence of light. The light source was provided by a solar simulator, and the light emitted by the light source conformed to the AM 1.5G standard solar spectrum.

### 2. Method for testing LUMO energy level

The test was carried out by ultraviolet photoelectron spectroscopy (UPS).

The material required to be characterized was spin-coated on a cleaned glass piece and annealed to obtain the film of this material, which was used directly for testing.

HOMO and LUMO energy levels can be obtained from Fermi edge and cutoff edge, respectively.

**Table 1**

| No. | Material in electron transport layer | LUMO energy level of Perovskite layer (eV) | LUMO energy level of Electron transport layer (eV) | Difference between LUMO energy levels of perovskite layer and electron transport layer | Photon-to-electron conversion efficiency on Day 3 | Photon-to-electron conversion efficiency on Day 30 |
|---|---|---|---|---|---|---|
| Comparative Embodiment 1 | C60 | -4.02 | -4.02 | 0.00 | 18.21% | 17.88% |
| Comparative Embodiment 2 | PCBM | -4.02 | -3.84 | 0.18 | 18.52% | 18.26% |
| Comparative Embodiment 3 | PCBM-0 | -4.02 | -3.88 | 0.14 | 18.62% | 18.35% |
| Comparative Embodiment 4 | PCBM | -4.02 | -3.84 | 0.18 | 18.42% | 18.50% |
| Embodiment 1 | PCBM-1 | -4.02 | -3.97 | 0.05 | 18.56% | 18.66% |
| Embodiment 2 | PCBM-2 | -4.02 | -3.91 | 0.11 | 18.73% | 19.01% |
| Embodiment 3 | PCBM-3 | -4.02 | -3.93 | 0.09 | 19.01% | 19.15% |
| Embodiment 4 | PCBM-4 | -4.02 | -3.92 | 0.10 | 19.32% | 19.33% |
| Embodiment 5 | PCBM-5 | -4.02 | -3.95 | 0.07 | 19.44% | 19.50% |
| Embodiment 6 | PCBM-6 | -4.02 | -3.98 | 0.04 | 19.50% | 19.65% |
| Embodiment 7 | PCBM-7 | -4.02 | -3.99 | 0.03 | 19.51% | 19.68% |

It can be seen from the data in Table 1 that:
compared with Comparative Embodiments 1-4, Embodiments 1-6 all achieved higher photon-to-electron conversion efficiency and better stability.

The LUMO energy level of the fullerene compound of the present application was increased when compared with fullerene C60. However, the LUMO energy level of the fullerene compound of the present application was decreased when compared with PCBM, which was achieved by introducing a specific functional group to PCBM. In addition, the compound of the present application had a smaller difference in HOMO energy level with perovskite, and was more suitable for matching with the perovskite formed by formamidine iodide (FAI), lead iodide (PbI₂), and methylamine chloride (MACl).

In Embodiment 2, the photon-to-electron conversion efficiency of the perovskite solar battery was 18.73% on day 3, and 19.01% on day 30. It can be seen that the photon-to-electron conversion efficiency has increased. This may be due to the fact that fullerene material of the present application will passivate the perovskite due to the introduction of a nitrogen-containing functional group (trimethylamino), and the perovskite itself will also have a self-repair process. When achieving a balance between the two, the efficiency will be improved. Embodiments 1-7 of the present application all illustrated this phenomenon.

In Comparative Embodiment 4, when PCBM was used as the electron transport layer, a passivation layer was used. It can be seen from the results that the photon-to-electron conversion efficiency and 30-day stability were improved by means of the passivation layer. However, this improvement still does not go as far as the present application can go. Compared with Comparative Embodiment 4, a higher photon-to-electron conversion efficiency and a better 30-day stability were achieved by Embodiments 1-7 of the present application in which a passivation layer (a passivation layer between the electron transport layer and the perovskite) was not used and a fullerene derivative useful for both transporting electrons and passivating perovskite described in the present application was used as an electron transport layer.

Compared with Comparative Embodiment 2, a benzene ring was introduced to the compound used in the electron transport layer of Embodiment 4 (benzene in Comparative Embodiment 2 vs. naphthalene in Embodiment 4), which not only greatly improved the photon-to-electron conversion efficiency of the perovskite solar batteries, but also significantly improved the long-term stability of the solar batteries.

By comparing Embodiment 4 introducing one compound of formula (1), Embodiment 5 introducing two compounds of formula (1), and Embodiment 7 introducing three compounds of formula (1), it can be seen that the higher the number of compounds of formula (1) as introduced, the better the photon-to-electron conversion efficiency and long-term stability, which may be due to a better matching between the energy levels of the electron transport layer and the perovskite layer using this fullerene derivative after the introduction of the above compounds.

It should be noted that the present application is not limited to the embodiments above. The above embodiments are merely exemplary, and embodiments having substantially the same technical idea and the same effects within the scope of the technical solutions of the present application are all included in the technical scope of the present application. In addition, without departing from the scope of the subject matter of the present application, various modifications applied to the embodiments that can be conceived by those skilled in the art, and other modes constructed by combining some of the constituent elements of the embodiments are also included in the scope of the present application.

## Claims

1. A fullerene derivative having a C60 fullerene group and a group of a compound of formula (1), formula (2) and/or formula (3) attached thereto, wherein the structural formulas of the compounds of formula (1), formula (2), and formula (3) are as follows: 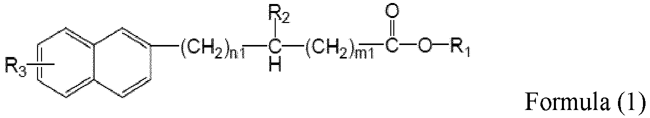 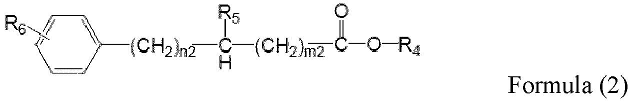 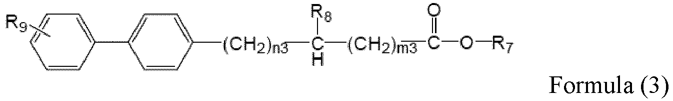 wherein
R₁, R₄, and R₇ are each independently selected from R', a phenyl group, a naphthyl group, a biphenyl group, R' substituted with a halogen, a phenyl group substituted with a halogen, a naphthyl group substituted with a halogen, a biphenyl group substituted with a halogen, a nitrogen-containing group, R' substituted with a nitrogen-containing group, a phenyl group substituted with a nitrogen-containing group, a naphthyl group substituted with a nitrogen-containing group or a biphenyl group substituted with a nitrogen-containing group;
R₂, R₅, and R₈ are each independently selected from hydrogen or R';
R₃, R₆, and R₉ are each independently selected from hydrogen, halogen, -O-R, a nitrogen-containing group, -OH, -OR, -NHCOR, -OCOR, -R, -CH₂COOH, a phenyl group or a naphthyl group;
the nitrogen-containing group is -N(R)₂, -NHR, -NH₂, a trimethylamine group, a triethylamine group or a tripropylamine group;
R' is an alkyl group with 1-5 carbon atoms;
R is an alkyl group with 1-10 carbon atoms;
n1, n2, n3, m1, m2, and m3 are each independently an integer in the range of 0-10, optionally an integer in the range of 0-5, and n1 + m1 ≤ 10, n2 + m2 ≤ 10, and n3 + m3 ≤ 10.

2. The fullerene derivative according to claim 1, wherein
R₁ is selected from R';
R₂ and R₅ are hydrogen;
R₃ is hydrogen;
R₄ is selected from R', a phenyl group or a trimethylamine group;
R₆ is selected from hydrogen or halogen, optionally hydrogen or fluorine;
n1 + m1 ≤ 5; and
n2 + m2 ≤ 5.

3. The fullerene derivative according to claim 1 or 2, wherein each C60 fullerene group is attached with 1-3, optionally 2, groups selected from the groups of compounds of formula (1), formula (2) and/or formula (3).

4. The fullerene derivative according to any of claims 1-3, wherein the LUMO energy level of the fullerene derivatives ranges from -4.02 to -3.72 eV, optionally from -4.02 to -3.82 eV, and more optionally from -4.00 to -3.92 eV.

5. The fullerene derivative according to any of claims 1-4, wherein the fullerene derivative can have the structure of formula (4), formula (5), formula (6), formula (7), formula (8), formula (9), formula (10), formula (11) or formula (12): 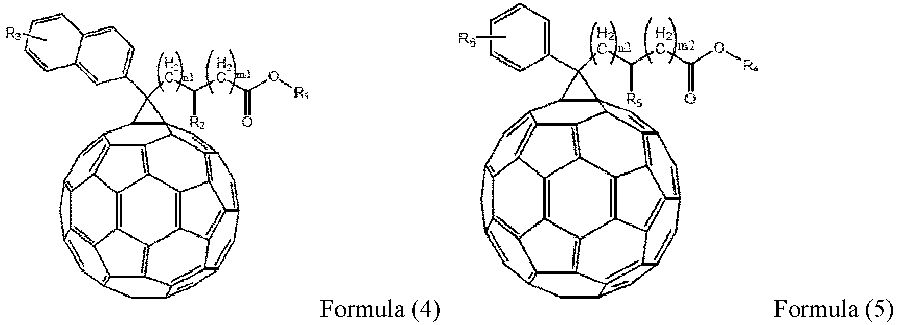 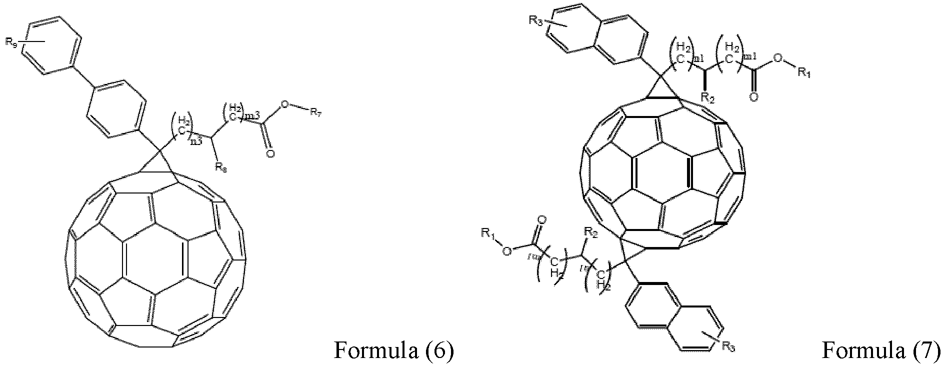 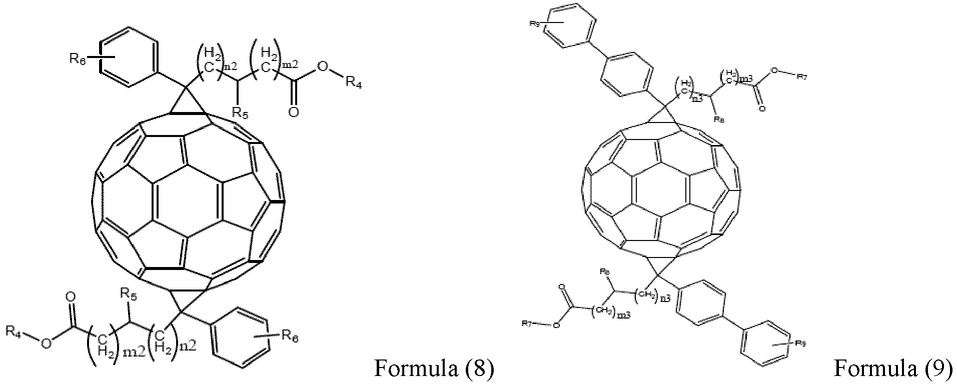 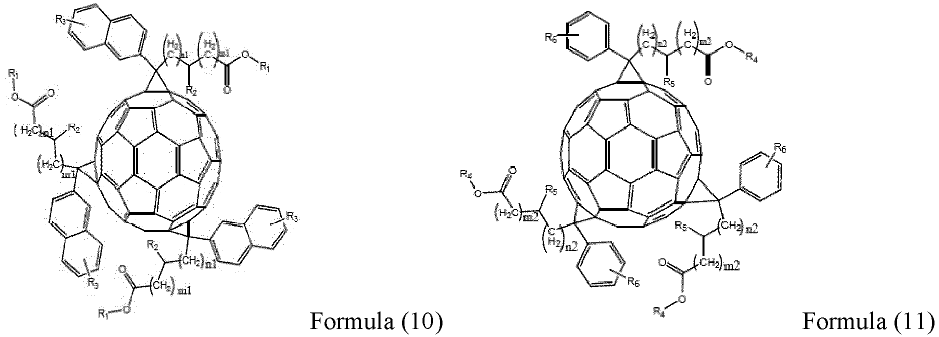 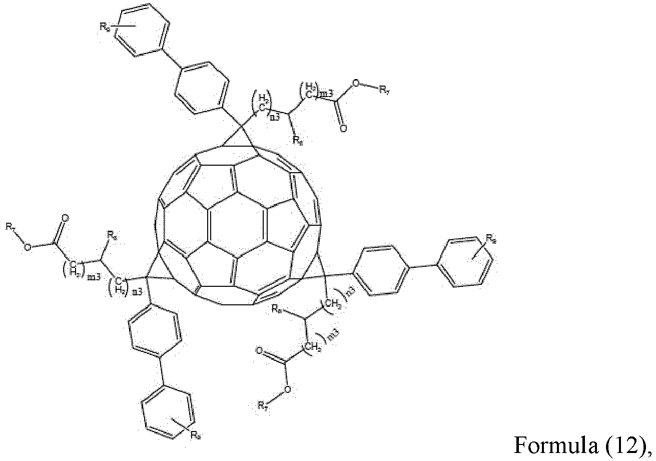 wherein R₁ to R₉, n1 to n3, and m1 to m3 are as defined in claim 1.

6. A perovskite solar battery comprising a conductive glass, a hole transport layer, a perovskite layer, an electron transport layer comprising a fullerene derivative and a back electrode, the fullerene derivative having a C60 fullerene group and a group of a compound of formula (1), formula (2) and/or formula (3) attached thereto, wherein the structural formulas of the compounds of formula (1), formula (2), and formula (3) are as follows: 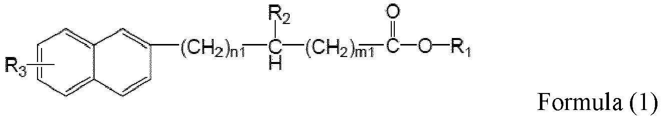 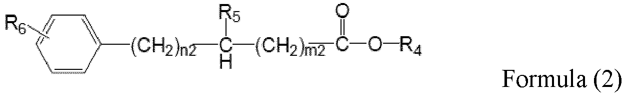 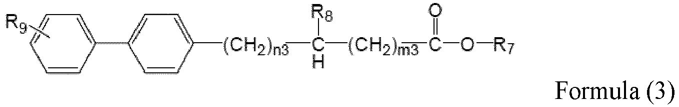 wherein
R₁, R₄, and R₇ are each independently selected from R', a phenyl group, a naphthyl group, a biphenyl group, R' substituted with a halogen, a phenyl group substituted with a halogen, a naphthyl group substituted with a halogen, a biphenyl group substituted with a halogen, a nitrogen-containing group, R' substituted with a nitrogen-containing group, a phenyl group substituted with a nitrogen-containing group, a naphthyl group substituted with a nitrogen-containing group or a biphenyl group substituted with a nitrogen-containing group;
R₂, R₅, and R₈ are each independently selected from hydrogen or R';
R₃, R₆, and R₉ are each independently selected from hydrogen, halogen, -O-R, a nitrogen-containing group, -OH, -OR, -NHCOR, -OCOR, -R, -CH₂COOH, a phenyl group or a naphthyl group;
the nitrogen-containing group is -N(R)₂, -NHR, -NH₂, a trimethylamine group, a triethylamine group or a tripropylamine group;
R' is an alkyl group with 1-5 carbon atoms;
R is an alkyl group with 1-10 carbon atoms;
n1, n2, n3, m1, m2, and m3 are each independently an integer in the range of 0-10, optionally an integer in the range of 0-5, and n1 + m 1 ≤ 10, n2 + m2 ≤ 10, and n3 + m3 ≤ 10.

7. The perovskite solar battery according to claim 6, wherein the perovskite solar battery is an inverted perovskite solar battery.

8. The perovskite solar battery according to claim 6 or 7, wherein the LUMO energy level of the electron transport layer is greater than or equal to that of the perovskite layer, and the difference between the LUMO energy levels of the electron transport layer and the perovskite layer is in the range of 0 to 0.2 eV.

9. The perovskite solar battery according to any one of claims 6-8, wherein no passivation layer is comprised between the perovskite layer and the electron transport layer.
